# EUROPEAN PATENT APPLICATION

(11) **EP 2 108 301 A1**
(43) Date of publication of application: **14.10.2009**
(21) Application number: 09151942.1
(22) Date of filing: 03.02.2009
(51) Int. Cl.: A61B 1/005

(54) **Endoscope, connection method of bending section and flexible section in endoscope.**

(30) Priority: 07.04.2008 JP 2008099609; 14.04.2008 US 44613 P
(71) Applicant: Olympus Corporation, Tokyo 151-0072 (JP)
(72) Inventor: Ito, Yoshiaki Olympus Intellectual Property Services Co., Ltd., Tokyo Tokyo 192-8512 (JP); Kitagawa, Hideya Olympus Intellectual Property Services Co., Ltd., Tokyo Tokyo 192-8512 (JP); Hashimoto, Hiroyuki Olympus Intellectual Property Services Co., Ltd., Tokyo Tokyo 192-8512 (JP)
(74) Representative: Winter, Brandl, Fürniss, Hübner Röss, Kaiser, Polte Partnerschaft Patent- und Rechtsanwaltskanzlei

(57) **Abstract**

An endoscope (1) includes a bending section (40) formed to be bendable by aligning a plurality of substantially cylindrical node rings (41, 51) and coupling the adjoining node rings (41, 51) to each other to allow them to swivel, a flexible section (20) directly coupled to the bending section (40) by fitting a distal end (20a) thereof into a proximal end side node ring (51) arranged on the most proximal end side of the bending section (40), an operation wire (90) that is inserted into the flexible section (20) and the bending section (40) and operates the bending section (40), and a wire guide section (91) that guides the operation wire (90) inserted into the flexible section (20) to the bending section (40) in the flexible section (20).

## Description

The present invention relates to an endoscope having a flexible section and a bending section, a connection method of a flexible section and a bending section in an endoscope, a production method of an endoscope provided for this connection method, an endoscope overtube having a flexible section and a bending section, a connection method of a flexible section and a bending section in an endoscope overtube, and a production method of an endoscope overtube provided for this connection method.

In general, an insertion section of an endoscope has a flexible (corrugated tube) section, a bending section coupled to a distal end of the flexible section, and a distal end hard section coupled to a distal end of the bending section. An operating section that operates the bending section is connected with a proximal end of the flexible section. An operation wire for bending operation is inserted into the insertion section. A distal end of this operation wire is coupled to the distal end hard section, and a proximal end of the operation wire is coupled to a bending operation mechanism provided in the operating section. Further, when the operation wire is pulled by an operation of the operating section through the bending operation mechanism, the bending section is remotely operated to bend.

The bending section is coupled to the flexible section through a coupling tube arranged between the bending section and the flexible section. The coupling tube is formed of a hard material. Furthermore, a guide coil is inserted into the flexible section. A distal end of this guide coil is fixed to the coupling tube and an inner surface of the coupling tube by, e.g., brazing or soldering. In the flexible section, the operation wire is inserted into the guide coil.

Moreover, on the other hand, when the insertion section of the endoscope is inserted into a body cavity, an endoscope overtube (which will be referred to as an overtube hereinafter) may be used in some cases. The insertion section is guided in the body cavity by the overtube. Some of the overtubes have an insertion section similar to the insertion section of the endoscope. The insertion section of the overtube has a flexible section and a bending section. The bending section and the flexible section of the overtube are coupled to each other through a coupling tube in the same way that the bending section and the flexible section of the endoscope are coupled to each other. It should be noted that the overtube is the same as the endoscope in that a guide coil or an operation wire for the bending section are arranged, an operating section is provided as in the endoscope, or the bending section is bent when the operation wire is pulled by the operating section.

Jpn. Pat. Appln. KOKAI Publication No. 2001-190495 discloses an endoscope apparatus in which a distal end portion of a coil that guides an operation wire is connected compact with a connection tube that connects a bending section with a flexible section while assuring connection strength.

Additionally, Jpn. Pat. Appln. KOKAI Publication No. 2007-252560 discloses an insertion section of an endoscope having a structure where a flexible tube skeleton in which a plurality of short cylindrical joint rings are coupled to each other through a coupling shaft to allow them to swivel in place of spiral tubes is used and a flexible envelope is formed by extrusion molding, the insertion section of the endoscope enabling smoothly coupling an insertion section flexible tube to a bending section without increasing a length of a hard section of a connecting section for the insertion section flexible tube and the bending section or locally producing a thin-walled portion in the flexible envelope formed by extrusion molding.

However, in the endoscope, when the coupling tube along the endoscope longitudinal axis (insertion direction of endoscope) is long, the length of the coupling portion (hard region) for a bending tube and a flexible tube is increased. This coupling portion does not bend. As a result, when the coupling portion is long, a minimum bending radius in the insertion section is not reduced, and the insertion section is not smoothly inserted into a body cavity having a complex shape. Therefore, it is preferable for the coupling portion for the bending section and the flexible section to be shorter.

As methods for reducing the length of the coupling portion, Jpn. Pat. Appln. KOKAI Publication No. 2001-190495 and Jpn. Pat. Appln. KOKAI Publication No. 2007-252560 are disclosed. However, in Jpn. Pat. Appln. KOKAI Publication No. 2001-190495 and Jpn. Pat. Appln. KOKAI Publication No. 2007-252560, a member serving as the coupling tube that couples the bending section to the flexible section is required. Therefore, the coupling portion is not shorter than this coupling tube.

Further, it is also preferable for the coupling portion for the bending section and the flexible section in the overtube to be shorter as in the case of the coupling portion for the bending section and the flexible section in the endoscope.

The present invention provides an endoscope that can shorten a coupling portion for a bending section and a flexible section and easily couple the bending section to the flexible section, a connection method of a flexible section and a bending section in an endoscope, a production method of an endoscope provided for this connection method, an endoscope overtube that can shorten a coupling portion for a bending section and a flexible section and easily couple the bending section to the flexible section, a connection method of a flexible section and a bending section in an endoscope overtube, and a production method of an endoscope overtube provided for this connection method.

The present invention provides an endoscope comprising: a bending section formed to be bendable by aligning a plurality of substantially cylindrical node rings and coupling the adjoining node rings to each other to allow them to swivel; a flexible section directly coupled to the bending section by fitting a distal end thereof into a proximal end side node ring arranged on the most proximal end side of the bending section; an operation wire that is inserted into the flexible section and the bending section and operates the bending section; and a wire guide section that guides the operation wire inserted into the flexible section to the bending section in the flexible section.

The present invention provides a connection method of a flexible section and a bending section in an endoscope which comprises: a flexible section; a bending section formed to be bendable by aligning a plurality of substantially cylindrical node rings and coupling the adjoining node rings to each other to allow them to swivel; an operation wire that is inserted into the flexible section and the bending section and operates the bending section; and a wire guide section that is arranged in the flexible section and guides the operation wire inserted into the flexible section to the bending section, the method comprising: a first step of fitting a distal end of the flexible section in a proximal end side node ring arranged on the most proximal end side of the bending section to directly couple the bending section and the flexible section to each other; and a second step of fixing the wire guide section on an inner peripheral surface of the distal end or an inner peripheral surface of the proximal end side node ring.

The present invention provides a production method of an endoscope comprising the connection method of a flexible section and a bending section in an endoscope.

The present invention provides an endoscope overtube comprising: a bending section formed to be bendable by aligning a plurality of substantially cylindrical node rings and coupling the adjoining node rings to each other to allow them to swivel; a flexible section directly coupled to the bending section by fitting a distal end thereof into a proximal end side node ring arranged on the most proximal end side of the bending section; an operation wire that is inserted into the flexible section and the bending section and operates the bending section; and a wire guide section that guides the operation wire inserted into the flexible section to the bending section in the flexible section.

The present invention provides a connection method of a flexible section and a bending section in an endoscope overtube which comprises: a flexible section; a bending section formed to be bendable by aligning a plurality of substantially cylindrical node rings and coupling the adjoining node rings to each other to allow them to swivel; an operation wire that is inserted into the flexible section and the bending section and operates the bending section; and a wire guide section that is arranged in the flexible section and guides the operation wire inserted into the flexible section to the bending section, the method comprising: a first step of fitting a distal end of the flexible section in a proximal end side node ring arranged on the most proximal end side of the bending section to directly couple the bending section and the flexible section to each other; and a second step of fixing the wire guide section on an inner peripheral surface of the distal end or an inner peripheral surface of the proximal end side node ring.

The present invention provides a production method of an endoscope comprising the connection method of a flexible section and a bending section in an endoscope overtube.

The invention can be more fully understood from the following detailed description when taken in conjunction with the accompanying drawings, in which:
FIG. 1 is a schematic structural view of an endoscope according to a first embodiment;
FIG. 2 is a cross-sectional view of a bending section and a flexible section coupled to each other taken along line A-A in FIG. 1;
FIG. 3 is a cross-sectional view taken along line B-B in FIG. 2;
FIG. 4 is a view showing an alignment state of node rings in the bending section;
FIG. 5 is a perspective view of the node ring;
FIG. 6 is a perspective view of a proximal end side node ring arranged on the most proximal end side of the bending section;
FIG. 7 is a cross-sectional view showing a coupled state of the flexible section and the bending section covered with an envelope and a mesh tube;
FIG. 8A is an enlarged view showing a part around an opening portion depicted in FIG. 7 and also showing a fixed state of the envelope;
FIG. 8B shows a modification of the fixed state of the envelope;
FIG. 9 is a partially cross-sectional view of a proximal end side node ring and a flexible tube side hard section coupled to each other, showing a modification of the proximal end side node ring;
FIG. 10 is a cross-sectional view of a bending section and a flexible section coupled to each other, showing an arrangement position of an excess metal portion according to a second embodiment;
FIG. 11A is a perspective view of a flexible section seen from a flexible tube side hard section according to a third embodiment;
FIG. 11B is a cross-sectional view of a bending section and a flexible section coupled to each other in the third embodiment;
FIG. 12A is a partially cross-sectional view of a proximal end side node ring and a flexible tube side hard section coupled to each other, showing a modification of the flexible tube side hard section;
FIG. 12B is a partially cross-sectional view of the proximal end side node ring and the flexible tube side hard section coupled to each other, showing a modification of the flexible tube side hard section;
FIG. 12C is a partially cross-sectional view of the proximal end side node ring and the flexible tube side hard section coupled to each other, showing a modification of the flexible tube side hard section;
FIG. 13A is a schematic structural view of an overtube according to a fourth embodiment; and
FIG. 13B is a cross-sectional view of a bending section and a flexible section coupled to each other in the overtube.

Embodiments according to the present invention will now be explained hereinafter in detail with reference to the accompanying drawings.

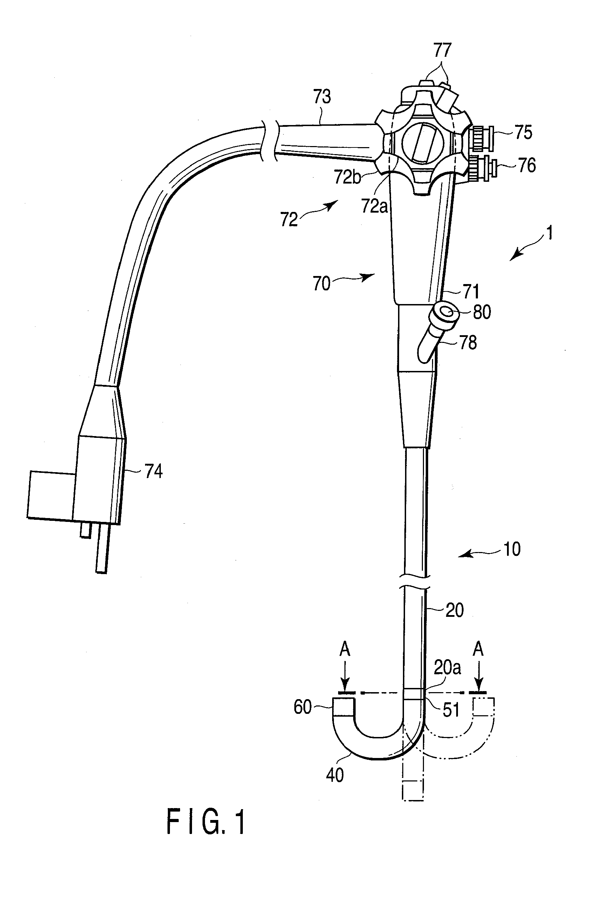

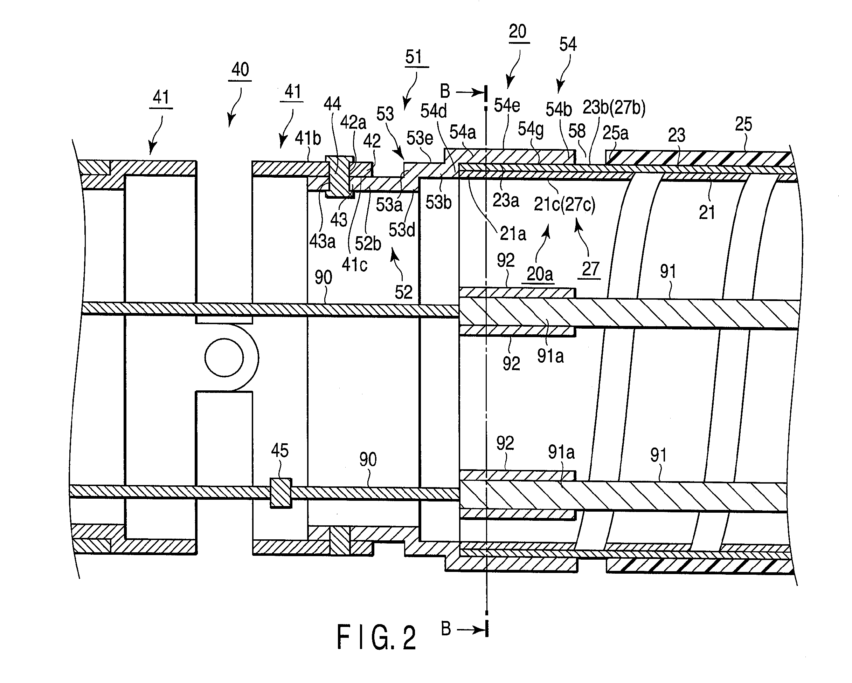

A first embodiment will be explained with reference to FIGS. 1 to 7 and FIG. 8A. It should be noted that tubular members and others are partially omitted in FIG. 2.

As shown in FIG. 1, an endoscope 1 has an elongated insertion section 10 that is inserted into, e.g., a body cavity of a patient and an operating section 70 that is coupled to a proximal end of the insertion section 10 and operates a later-explained bending section 40 of the insertion section 10.

In the operating section 70 are provided a grasping section 71 grasped by an operator and a bending operation knob 72 that bends the bending section 40.

A proximal end portion of a universal cord 73 is coupled to the grasping section 71. A connector section 74 connected with a non-illustrated light source device or a video processor is coupled to a distal end portion of this universal cord 73.

In a bending operation knob 72 are provided a lateral bending operation knob 72a that operates the bending section 40 to laterally bend and a vertical bending operation knob 72b that operates the bending section 40 to vertically bend. A non-illustrated bending operation mechanism for a lateral direction that is driven by the lateral bending operation knob 72a is connected with the lateral bending operation knob 72a. Further, a non-illustrated bending operation mechanism for a vertical direction that is driven by the vertical bending operation knob 72b is connected with the vertical bending operation knob 72b. The vertical bending operation mechanism and the lateral bending operation mechanism are arranged in the operating section 70 and connected with a proximal end of a later-explained operation wire 90.

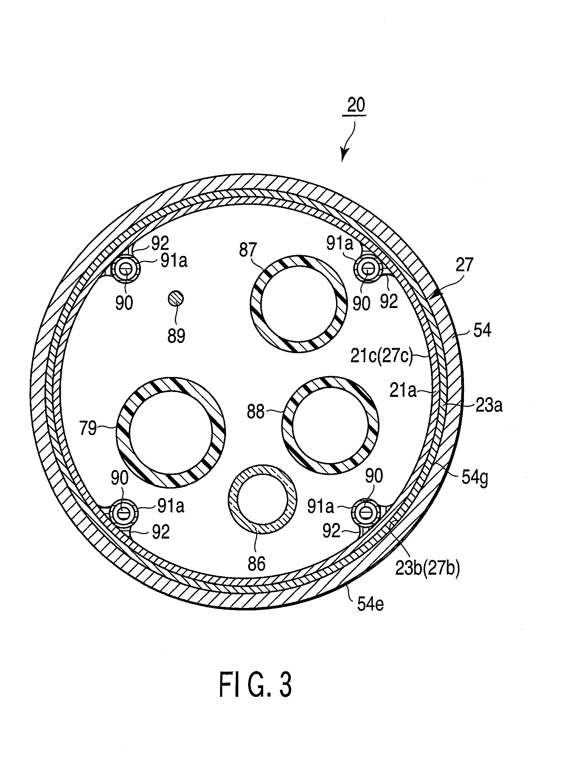

It should be noted that a suction button 75, an air supply/water supply button 76, various kinds of buttons 77 for endoscopic imaging, and a surgical instrument insertion section 78 are provided to the operating section 70. A surgical instrument insertion opening 80 is provided in the surgical instrument insertion section 78. A proximal end portion of a later-explained surgical instrument insertion channel 79 depicted in FIG. 3 is coupled to the surgical instrument insertion opening 80. The surgical instrument insertion channel 79 is arranged in the insertion section 10. The surgical instrument insertion opening 80 is an insertion opening through which a non-illustrated endoscope surgical instrument is inserted into the surgical instrument insertion channel 79. The non-illustrated endoscope surgical instrument is inserted into the surgical instrument insertion channel 79 from the surgical instrument insertion opening 80 of the endoscope 1. The non-illustrated endoscope surgical instrument is pushed to a later-explained distal end hard section 60 of the insertion section 10, and then protruded from a non-illustrated distal end opening portion of the surgical instrument insertion channel 79 provided in the distal end hard section 60.

The insertion section 10 has a flexible section (corrugated tube section) 20, the bending section 40, and the distal end hard section 60 in the mentioned order from the operating section 70 side. In more detail, the operating section 70 is coupled to a proximal end of the elongated flexible section 20. A distal end 20a of the flexible section 20 is directly coupled to a proximal end of the bending section 40. In more detail, the flexible section 20 is directly coupled to the bending section 40 by fitting the distal end 20a thereof into a later-explained node ring 51 arranged on the most proximal end side of the bending section 40. A distal end of the bending section 40 is coupled to a proximal end of the distal end hard section 60.

The flexible section 20 is, e.g., hollow. In more detail, as shown in FIGS. 2 and 3, the flexible section 20 has a flex 21, a mesh-like braid 23 that is arranged on an outer side of this flex 21 and covers the flex 21, and an envelope 25 that is arranged on an outer side of this braid 23 and covers the braid 23. The flexible section 20 has a three-layer structure including the flex 21, the braid 23, and the envelope 25.

The flex 21 is obtained by spirally molding, e.g., a strip-like thin plate material formed of stainless steel or the like into substantially a cylinder. The flex 21 is, e.g., a thin-walled metal spiral tube.

The braid 23 is obtained by forming, e.g., a braided wire bundle including a plurality of wire bundles of stainless steel or the like into substantially a cylinder. The braid 23 is, e.g., a mesh tube.

The envelope 25 is formed into substantially a cylinder to cover the outer side of the braid 23 with a resin material having flexibility, e.g., a rubber material. A thickness of the envelope 25 is substantially equal to a thickness of a proximal end portion 54 of the later-explained node ring 51, and an outer diameter and an inner diameter of the envelope 25 are substantially equal to an outer diameter and an inner diameter of the proximal end portion 54.

A distal end 21a of the flex 21 and a distal end 23a of the braid 23 are extended to a distal end side of the flexible section 20 (bending section 40 side, node ring 51 side) from a distal end 25a of the envelope 25 by the same desired length. Therefore, an outer peripheral surface 23b of the distal end 23a is exposed. It should be noted that the distal end 21a is not exposed since it is covered with the distal end 23a. In other words, the distal end 25a is shorter than the distal end 21a and the distal end 23a. As explained above, the distal end 21a and the distal end 23a serve as the distal end 20a of the flexible section 20.

The distal end 21a and the distal end 23a are secured to be integrated in advance by, e.g., soldering. When the distal end 21a and the distal end 23a are integrated, a hard flexible tube side hard section 27 having rigidity is formed. That is, the distal end 20a becomes hard, and the flexible section 20 has at the distal end 20a the flexible tube side hard section 27 having rigidity which is integrated with the distal end 20a. Further, the flexible tube side hard section 27 has the flex 21 and the braid 23 that covers the flex 21.

It should be noted that an outer peripheral surface 27b of the flexible tube side hard section 27 is the outer peripheral surface 23b. An inner peripheral surface 27c of the flexible tube side hard section 27 is an inner peripheral surface 21c of the distal end 21a. The flexible tube side hard section 27 is directly fitted in the proximal end portion 54 of the node ring 51 and bonded and fixed to the proximal end portion 54 by, e.g., a non-illustrated adhesive. As a result, the flexible section 20 and the bending section 40 are directly coupled to each other. As explained above, the flexible tube side hard section 27 is a coupling section in the flexible section 20 where the flexible section 20 is directly coupled to the bending section 40 (node ring 51).

That is, the bending section 40 (node ring 51) and the flexible section 20 are not coupled to each other by a coupling member arranged between the bending section 40 and the flexible section 20, but they are coupled to each other without using the coupling member. Furthermore, the bending section 40 (node ring 51) and the flexible section 20 are not coupled to each other in a state where the coupling member is arranged to at least one of the bending section 40 and the flexible section 20.

It should be noted that an outer diameter of the flexible tube side hard section 27 is an outer diameter of the braid 23 and it is substantially equal to an inner diameter of the proximal end portion 54. Therefore, when the flexible tube side hard section 27 is directly fitted in the proximal end portion 54 of the node ring 51, the outer peripheral surface 23b as the outer peripheral surface 27b comes into contact with an inner peripheral surface 54g of the proximal end portion 54 to be secured and fixed to the inner peripheral surface 54g.

An internal structure of the flexible section 20 will now be briefly explained with reference to FIG. 3.

As shown in FIG. 3, into the flexible section 20 are inserted the surgical instrument insertion channel 79, a light guide fiber 86, an air supply tube 87, a water supply tube 88, a cable 89 such as a signal line, operation wires 90, wire guide sections 91 that guide and protect the operation wires 90, and others.

Tubular members, e.g., the surgical instrument insertion channel 79, the light guide fiber 86, the air supply tube 87, the water supply tube 88, the cable 89, and others are components provided in the endoscope 1. These tubular members are inserted into the bending section 40 from the operating section 70 side through the flexible section 20 and coupled to the proximal end of the distal end hard section 60.

The four operation wires 90 operate the entire bending section 40 to bend in four directions, i.e., the upward, downward, leftward, and rightward directions, respectively.

The four operation wires 90 are components provided in the endoscope 1 like the tubular members. Distal ends of the four operation wires 90 are inserted into the bending section 40 from the operating section 70 through the flexible section 20 to be coupled to the proximal end of the distal end hard section 60. Proximal ends of the two operation wires 90 that bend the bending section 40 in the left and right directions in these four operation wires 90 are coupled to the above-explained bending operation mechanism for the lateral direction in the operating section 70. Moreover, proximal ends of the two operation wires 90 that bend the bending section 40 in the upward and downward directions are coupled to the bending operation mechanism for the vertical direction in the operating section 70.

By swiveling the lateral bending operation knob 72a and the vertical bending operation knob 72b, the respective operation wires 90 are driven to be pulled through the bending operation mechanism for the lateral direction and the bending operation mechanism for the vertical direction. As a result, the bending section 40 is remotely operated to bend from a regular straight state where the bending section 40 is straight and has a bending angle of 0° (e.g., a non-bent state indicated by a line of alternate long and short dashes depicted in FIG. 1) to a bent state where the same is bent in the upward, downward, leftward, and rightward directions at an arbitrary bending angle (e.g., a state indicated by a solid line or a chain double-dashed line depicted in FIG. 1).

Each wire guide section 91 guides each operation wire 90 extended from the operating section 70 and inserted into the flexible section 20 to the bending section 40 in the flexible section 20 as shown in FIG. 2.

One operation wire 90 is inserted into one wire guide section 91. In this embodiment, since the number of the operation wires 90 is four, the four wire guide sections 91 are provided.

A distal end 91a of the wire guide section 91 is fixed to the inner peripheral surface 21c as the inner peripheral surface 27c as shown in FIGS. 2 and 3. In more detail, an excess metal portion 92 is previously formed on the inner peripheral surface 27c by, e.g., brazing or soldering. It is preferable for the excess metal portion 92 to be formed on the most distal end side of the inner peripheral surface 27c. When the distal end 91a is secured to the excess metal section 92, it is fixed to the inner peripheral surface 27c, i.e., the distal end 20a. As explained above, the wire guide section 91 is arranged in the flexible section 20.

It should be noted that the same number of the excess metal portions 92 as the number of the wire guide sections 91 are provided and one wire guide section 91 is fixed to one excess metal portions 92. In this embodiment, since the number of the wire guide sections 91 is four, the four excess metal portions 92 are provided. It is preferable for the excess metal sections 92 to be apart from each other in a circumferential direction at substantially 90°. As a result, distal ends 91a are fixed to be apart from each other in the circumferential direction at substantially 90°.

A structure of the bending section 40 will now be explained.

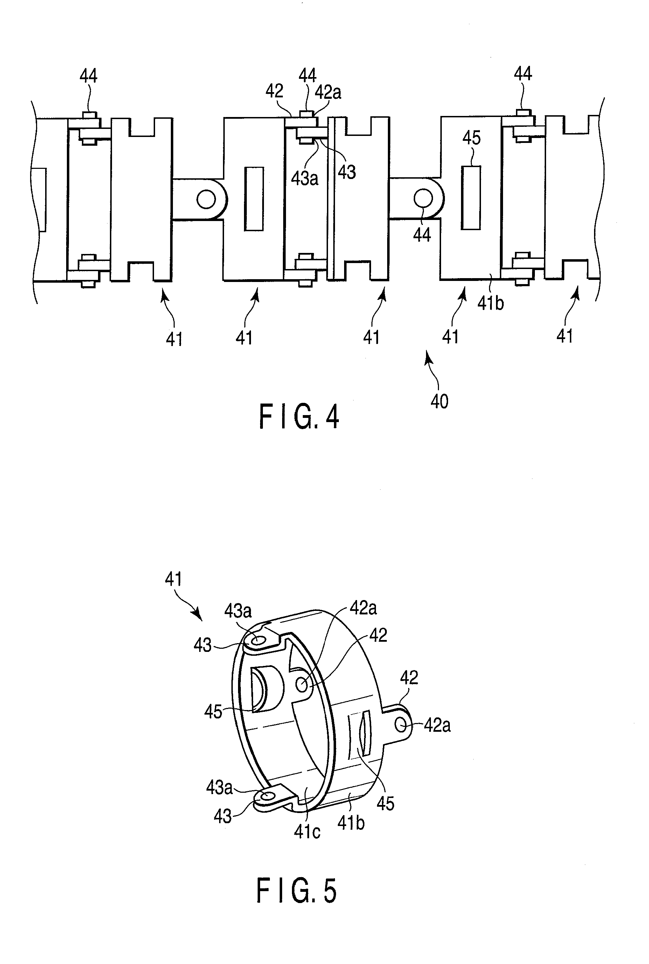

As shown in FIG. 4, in the bending section 40, a plurality of substantially cylindrical node rings 41 are aligned along the insertion axis (longitudinal axis) of the insertion section 10. The node rings 41 adjoin to each other (placed at front and rear positions along the insertion direction of the insertion section 10) are coupled to each other to allow them to swivel by spindle sections (e.g., rivets 44 as later-explained swiveling members). The node rings 41 are coupled to each other in this manner to form the bending section 40. The node ring 41 is formed of a hard material, e.g., a metal.

It should be noted that the distal end hard section 60 is coupled to the node ring 41 arranged at a position closest to the distal end hard section 60. Moreover, the flexible section 20 is coupled to the node ring 51 arranged at a position closest to the flexible section 20 side as shown in FIG. 2 (particulars will be explained later). The node ring 51 serves as a proximal end side node ring arranged on the most proximal end side of the bending section 40.

A structure of the node ring 41 will now be explained.

This node ring 41 means a node ring arranged at a position closer to the distal end side as compared with the node ring 51. As shown in FIG. 5, each node ring 41 is substantially a cylinder. The node ring 41 is molded as, e.g., a sheet metal pressed part or a cast.

Two protruding pieces (rear hinge bases) 42 are arranged on a rear end portion side (the right-hand side in FIG. 5) of the node ring 41. The protruding piece 42 is formed by protruding a part of the node ring 41 toward the rear side (proximal end side of the bending section 40). Additionally, a through-hole 42a is formed in the protruding piece 42. The two protruding pieces 42 are arranged to be apart from each other in the circumferential direction at substantially 180°.

Further, two protruding pieces (front hinge bases) 43 are arranged on a front end portion side (left-hand side in FIG. 5) of the node ring 41. The protruding piece 43 is formed by protruding a part of the node ring 41 toward the front side (distal end side of the bending section 40). A step substantially corresponding to a board thickness of the protruding piece 42 is provided to the protruding piece 43. Furthermore, a through-hole 43a is formed in the protruding piece 43. The two protruding pieces 43 are arranged to be apart from each other in the circumferential direction at substantially 180°.

The front two protruding pieces 43 and the rear two protruding pieces 42 are arranged at positions to be apart from each other in the circumferential direction at substantially 90°.

Moreover, two wire receivers 45 are inwardly formed on an inner peripheral wall portion of the node ring 41. The wire receiver 45 is formed at a position close to the front side in the axial direction of the node ring 41 as compared with the protruding piece 42. The two wire receivers 45 are arranged to be apart from each other in the circumferential direction at substantially 180°.

Each wire receiver 45 is formed by cutting, bending, and raising a part of a circumferential wall portion of the node ring 41 to be protruded from an outer peripheral surface 41b side toward an inner peripheral surface 41c side of the node ring 41 by press working. The operation wire 90 for the vertical direction and the operation wire 90 for the lateral direction are inserted into the wire receivers 45 and held in the wire receivers 45. Each wire receiver 45 is a holding section that holds the operation wire 90 in the bending section 40, and serves as an insertion section into which the operation wire 90 is inserted.

The four operation wires 90 held in the wire receivers 45 are arranged in the bending section 40. The operation wires 90 are arranged along the inner peripheral surface 41c side. The distal end portions of these four operation wires 90 are fixed to the distal end hard section 60 by, e.g., silver brazing or soldering as explained above. It should be noted that the distal end portions of the operation wires 90 may be fixed to the node ring 41 placed at the position closest to the distal end hard portion 60.

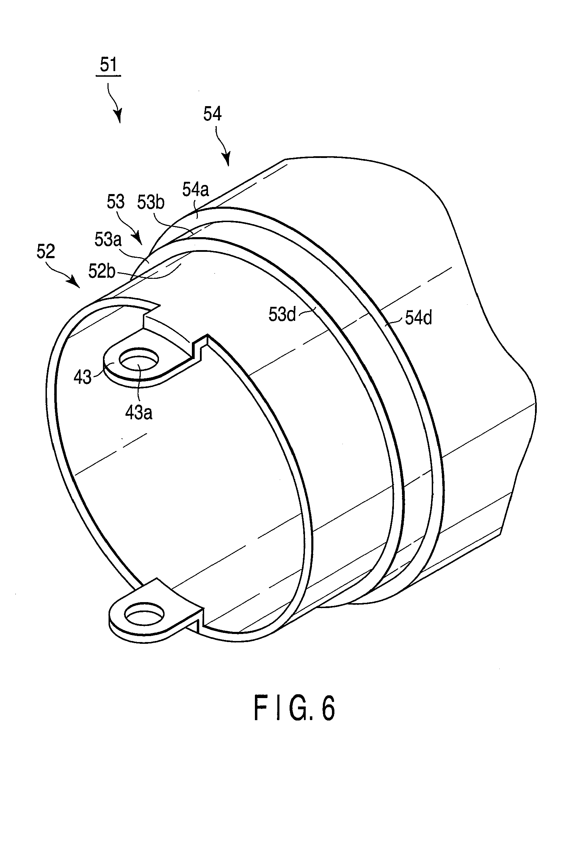

A structure of the node ring 51 will now be explained with reference to FIGS. 2 and 6.

The structure of the node ring 51 on the front end portion side is substantially equal to a structure of the node ring 41 on the front end portion side, but structures other than this is different. The node ring 51 is included in the bending section 40.

The node ring 51 includes a substantially cylindrical distal end portion 52 corresponding to the front end portion side of the node ring 51, a substantially cylindrical middle end portion 53 that is continuous with a proximal end 52b of the distal end portion 52 at a distal end 53a thereof, and a substantially cylindrical proximal end portion 54 that is continuous with the proximal end 52b of the middle end portion 53 at a distal end 54a thereof.

The distal end portion 52 has protruding pieces 43 like the node ring 41. An outer diameter of the distal end portion 52 is substantially equal to an inner diameter of the node ring 41. Therefore, each protruding piece 43 comes into contact with the inner peripheral surface 41c. The distal end portion 52 is coupled to the neighboring node ring 41 in the same way that the neighboring node rings 41 are coupled to each other.

A diameter of the middle end portion 53 is larger than that of the distal end portion 52 in a radial direction. In more detail, the middle end portion 53 has a step portion 53d having a thickness that is close to a board thickness of the protruding piece 42 at the distal end 53a that is a portion provided to be continuous with the distal end portion 52. This step portion 53d is arranged over the entire circumference of the distal end 53a of the substantially cylindrical middle end portion 53 in the middle end portion 53. A diameter of the step portion 53d is increased toward the outer peripheral side in the radial direction.

An inner diameter and an outer diameter of the middle end portion 53 are substantially equal to an inner diameter and an outer diameter of the node ring 41.

A diameter of the proximal end portion 54 is larger than the middle end portion 53 in the radial direction. In more detail, the proximal end portion 54 has a step portion 54d having a thickness that is close to a board thickness of the distal end 21a and the distal end 23a (flexible tube side hard section 27) at the distal end 54 that is a portion provided to be continuous with the middle end portion 53. This step portion 54d is arranged over the entire circumference of the distal end 54a of the substantially cylindrical proximal end portion 54 in the proximal end portion 54. A diameter of the step portion 54d is increased toward the outer peripheral side in the radial direction.

The step portion 54d is an abutting portion on which the distal end 21a and the distal end 23a (flexible tube side hard section 27) abut, and it is also a preventing portion that prevents the distal end 21a and the distal end 23a (flexible tube side hard section 27) from being fitted in toward the distal end side of the endoscope 1 (distal end portion 52 or the middle end portion 53 corresponding to the distal end side of the node ring 51). That is, the step portion 54d prevents the distal end 21a and the distal end 23a (flexible tube side hard section 27) from being fitted in to reach the distal end side of the endoscope 1 beyond this abutting position (step portion 54d) when the distal end 21a and the distal end 23a (flexible tube side hard section 27) abut the step portion 54d.

As explained above, the node ring 51 has at the proximal end portion 54 the step portion 54d with which the flexible tube side hard section 27 comes into contact when the flexible tube side hard section 27 is fitted in the node ring 51.

Further, each of the inner diameter and the outer diameter of the proximal end portion 54 is larger than the outer diameter of the node ring 41. Furthermore, the inner diameter of the proximal end portion 54 is larger than the outer diameter of the middle end portion 53 and also larger than the outer diameter of the node ring 41. At this time, wall thicknesses of the middle end portion 53 and the proximal end portion 54 may be fixed or may be different from each other. It should be noted that it is preferable for the wall thicknesses of the distal end portion 52, the middle end portion 53, and the proximal end portion 54 to be fixed.

The inner diameter of the proximal end portion 54 is substantially equal to the outer diameter of the flexible tube side hard section 27. Furthermore, the flexible tube side hard section 27 is directly fitted in the proximal end portion 54. At this time, the flexible tube side hard section 27 comes into contact with the step portion 54d and the inner peripheral surface 54g of the proximal end portion 54 comes into contact with the outer peripheral surface 27b to be bonded and fixed (coupled) to each other by, e.g., a non-illustrated adhesive. As a result, the proximal end portion 54 is bonded and fixed to the flexible tube side hard section 27. Therefore, the flexible section 20 and the bending section 40 are directly coupled to each other. It should be noted that a solder or a laser may be used as the coupling method, for example.

As explained above, the flexible tube side hard section 27 is directly fitted into the proximal end portion 54, and the proximal end portion 54 is bonded and fixed to the flexible tube side hard section 27 by, e.g., a non-illustrated adhesive. That is, the proximal end portion 54 is a coupling portion in the bending section 40 where the bending section 40 is directly coupled to the flexible section 20.

It should be noted that the inner diameter and the outer diameter of the proximal end portion 54 are substantially equal to the outer diameter and the inner diameter of the envelope 25. That is, a thickness of the proximal end portion 54 is substantially equal to a thickness of the envelope 25. Moreover, when the flexible tube side hard section 27 is directly fitted into the proximal end portion 54, the proximal end 54b of the proximal end portion 54 does not come into contact with the distal end 25a. An opening portion 58 having a desired length in the insertion direction of the insertion section 10 is formed between the proximal end 54b and the distal end 25a.

It should be noted that it is preferable for a length of the node ring 51 in the insertion direction of the insertion section 10 to be short in a state where a minimum bending radius of the insertion section 10 is maintained. As a result, a length of a non-bent portion can be reduced with a length of a coupling portion of the flexible section 20 and the bending section 40 in the insertion direction of the insertion section 10.

Additionally, it is preferable for a length of the proximal end portion 54 in the insertion direction of the insertion section 10 and a length of the flexible tube side hard section 27 in the insertion direction of the insertion section 10 to be short. As a result, a length of a non-bent portion can be reduced with a length of a coupling portion of the flexible section 20 and the bending section 40 in the insertion direction of the insertion section 10.

A coupling method for the node rings 41 will now be explained.

As shown in FIG. 4, the rivets 44 as swiveling members are inserted into the through-holes 42a and 43a in the protruding pieces 42 of the front node ring 41 and the protruding pieces 43 of the rear node ring 41. As a result, the front node ring 41 is coupled to the rear node ring 41 by, e.g., caulking one end of each rivet 44, and they are pivotally supported to swivel around each rivet 44. In this manner, a spindle section using the rivet 44 as a swiveling spindle is formed between the protruding piece 42 and the protruding piece 43.

In the bending section 40 according to this embodiment, the rivets 44 serving as the swiveling spindles that couple the plurality of node rings 41 to each other are alternately arranged to be alternated at substantially 90° between the respective node rings 41. As a result, the bending section 40 is configured to bend in the four directions, i.e., the upward, downward, leftward, and rightward directions when pushed or pulled by the operation wires 90.

Coupling the node ring 41 with the node ring 51 is substantially equivalent to coupling all the node rings 41 to each other, obviating explanation thereof.

A connection method of the flexible section 20 and the bending section 40 (flexible tube side hard section 27 and node ring 51) will now be explained.

The distal end 21a is covered with the distal end 23a, and the distal end 23a (outer peripheral surface 23b) is exposed without being covered with the envelope 25. In this state, the distal end 21a and the distal end 23a are secured in advance by, e.g., soldering. When they are secured, the distal end 21a is integrated with the distal end 23a, and the flexible tube side hard section 27 is formed at the distal end 20a of the flexible section 20. The flexible tube side hard section 27 is directly fitted into the proximal end portion 54. At this time, the flexible tube side hard section 27 comes into contact with the step portion 54d, and the outer peripheral surface 27b comes into contact with the inner peripheral surface 54g. The proximal end 54b does not come into contact with the distal end 25a, and the opening portion 58 is formed. Further, the outer peripheral surface 27b and the inner peripheral surface 54g which are in contact with each other are bonded and fixed to each other by, e.g., a non-illustrated adhesive. As a result, the flexible tube side hard section 27 is directly coupled to the proximal end portion 54. Therefore, the flexible section 20 and the bending section 40 are directly coupled to each other through the flexible tube side hard section 27 and the proximal end portion 54.

It should be noted that the flexible tube side hard section 27 as a coupling portion in the bonded and fixed flexible section 20 and the proximal end portion 54 as a coupling portion in the bending section 40 are coupling positions for the flexible section 20 and the bending section 40.

Furthermore, the distal end 91a is fixed on the inner peripheral surface 27c (inner peripheral surface 21c) by the excess metal portion 92. It should be noted that the distal end 91a may be fixed when the flexible tube side hard section 27 is formed, before the flexible tube side hard section 27 is directly fitted into the proximal end portion 54, or after the flexible tube side hard section 27 is coupled to the proximal end portion 54. The fixing means for the distal end 91a does not have to be restricted to the excess metal portion 92, and the distal end 91a may be fixed by, e.g., soldering, brazing, or a laser.

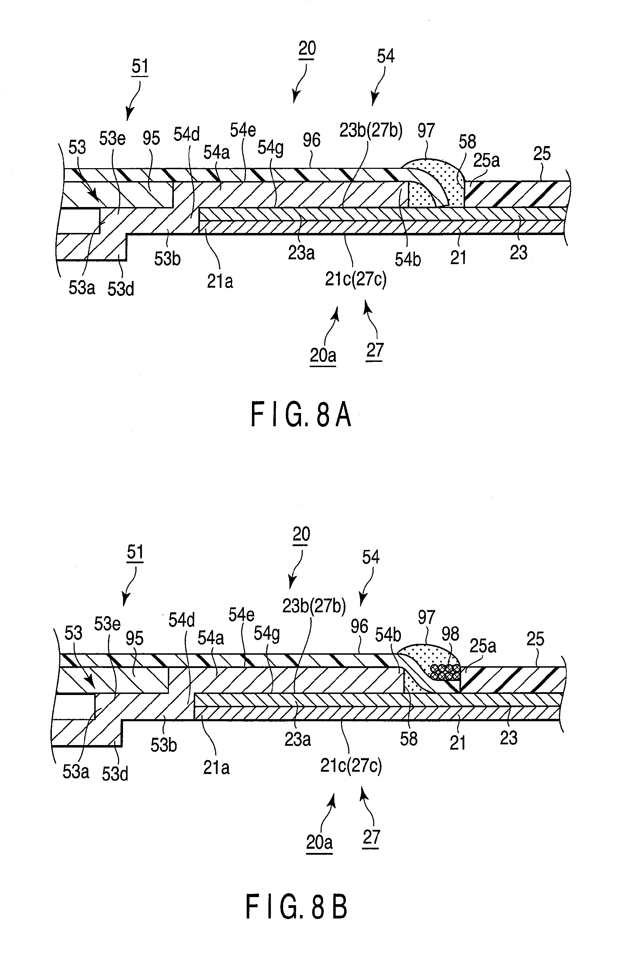

When the flexible section 20 is directly coupled to the bending section 40 and the distal end 91a is fixed to the flexible tube side hard section 27, the bending section 40 is covered with a braid (mesh tube) 95 that is equal or similar to the braid 23. Moreover, the braid 95 covers the outer peripheral surface 41b and the outer peripheral surface 53e of the middle end portion 53. Additionally, the braid 95 is covered with, e.g., a resin envelope 96 that is equal to the envelope 25. The envelope 96 covers the braid 95 and the outer peripheral surface 54e of the proximal end portion 54. Further, the envelope 96 is bonded by, e.g., an adhesive 97 in the opening portion 58 as shown in FIG. 8A.

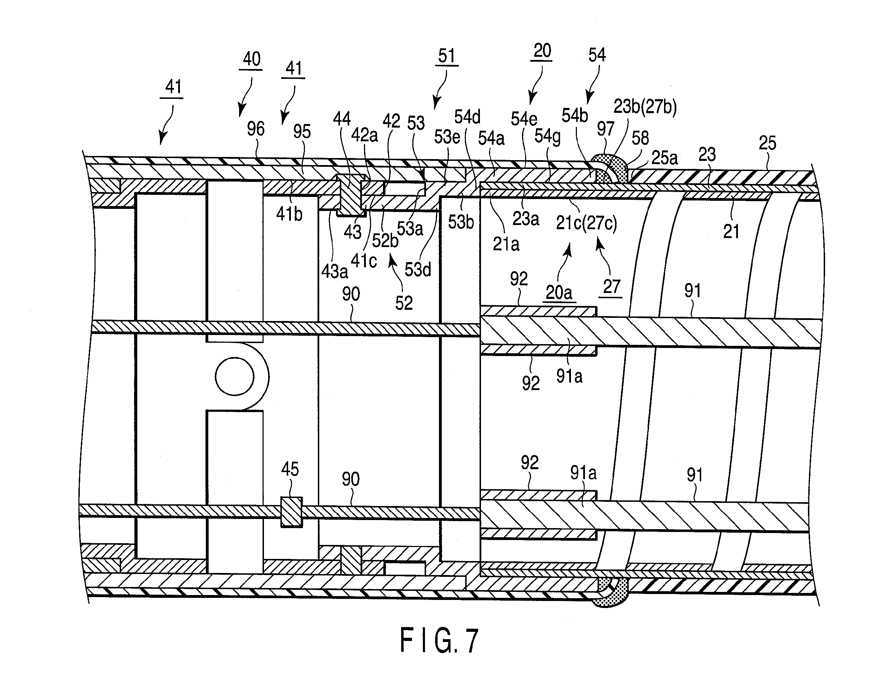

It should be noted that an outer peripheral surface of the adhesive 97 is raised to be higher than an outer periphery of the envelope 96 in this embodiment as shown in FIG. 7, but the present invention is not restricted to this conformation, and it may be formed to have substantially the same height as that of the outer periphery of the envelope 96. This structure can likewise be applied to the following different embodiment.

As explained above, in this embodiment, the flexible section 20 can be directly coupled to the bending section 40 by directly fitting the flexible tube side hard section 27 into the proximal end portion 54 without using a coupling member, e.g., a mouth ring that couples the flexible section 20 to the bending section 40. Therefore, in this embodiment, since the coupling member is not used, the length of the coupling portion coupling the flexible section 20 to the bending section 40 in the insertion direction of the insertion section 10 can be reduced by shortening the lengths of the flexible tube side hard section 27 and the proximal end portion 54 in the insertion direction of the insertion section 10.

Furthermore, in this embodiment, the length of the coupling portion as the non-bent (swiveling) portion can be reduced by shortening the length of the coupling portion in the insertion direction of the insertion section 10. As a result, when performing, e.g., an examination of the large intestine, since a minimum bending radius of the insertion section 10 can be decreased in this embodiment, contact of the insertion section 10 with respect to an organ can be reduced. As a result, this embodiment can provide a patient with a minimally invasive surgery.

Moreover, in this embodiment, the flexible tube side hard section 27 is directly fitted into the proximal end portion 54, and the proximal end portion 54 is bonded and fixed to the flexible tube side hard section 27 by, e.g., an adhesive, thereby readily coupling the flexible section 20 to the bending section 40.

Additionally, since this embodiment does not use the coupling member, the endoscope 1 can be provided at low cost.

Further, in this embodiment, the step portion 54d can prevent the flexible tube side hard section 27 from being fitted in to reach the distal end side in the insertion direction of the endoscope 1, and bringing the step portion 54d in contact with the flexible tube side hard section 27 enables providing an index which is used when coupling the flexible section 20 to the bending section 40.

Furthermore, in this embodiment, when the step portion 54d comes into contact with the flexible tube side hard section 27, the flexible tube side hard section 27 can be fitted in to reach the bending section 40 side, thereby further reducing the length of the coupling portion.

It should be noted that the proximal end portion 54 is cylindrical, but the present invention is not restricted thereto, and the proximal end portion 54 may have, e.g., a protruding piece like the protruding piece 42 so that it can be coupled to the flexible tube side hard section 27 by using this protruding piece.

Moreover, this embodiment can provide the connection method of the flexible section 20 and the bending section 40 and the production method of the endoscope 1 provided for this connection method.

It should be noted that the envelope 96 may be fixed in the opening portion 58 by using the adhesive 97 and a thread 98 as shown in FIG. 8B.

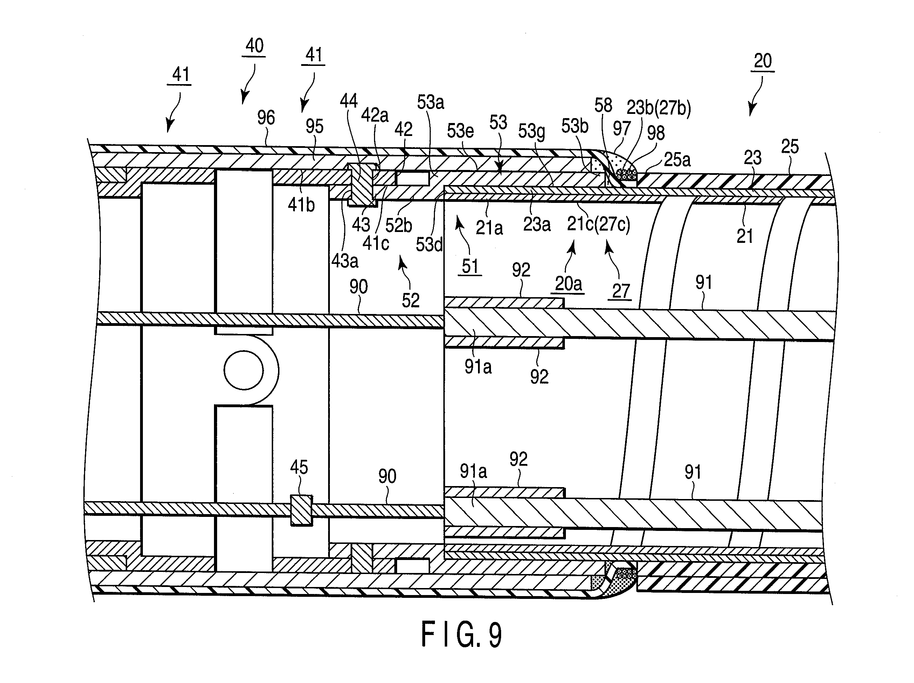

Additionally, the node ring 51 may have the distal end portion 52 and the middle end portion 53 alone as shown in FIG. 9. In this case, the inner diameter of the middle end portion 53 is substantially equal to the outer diameter of the flexible tube side hard section 27, and the flexible tube side hard section 27 is fitted into the middle end portion 53. That is, the middle end portion 53 serves as the proximal end portion. Further, the outer peripheral surface 27b (outer peripheral surface 23b) comes into contact with the inner peripheral surface 53g of the middle end portion 53. The opening portion 58 is formed in the proximal end 53b of the middle end portion 53 and the distal end 25a. The step portion 53d is an abutting portion on which the distal end 21a and the distal end 23a (flexible tube side hard section 27) abut, and it serves as a preventing portion that prevents the distal end 21a and the distal end 23a (flexible tube side hard section 27) to be fitted in to reach the distal end side (distal end portion 52 side) of the endoscope 1.

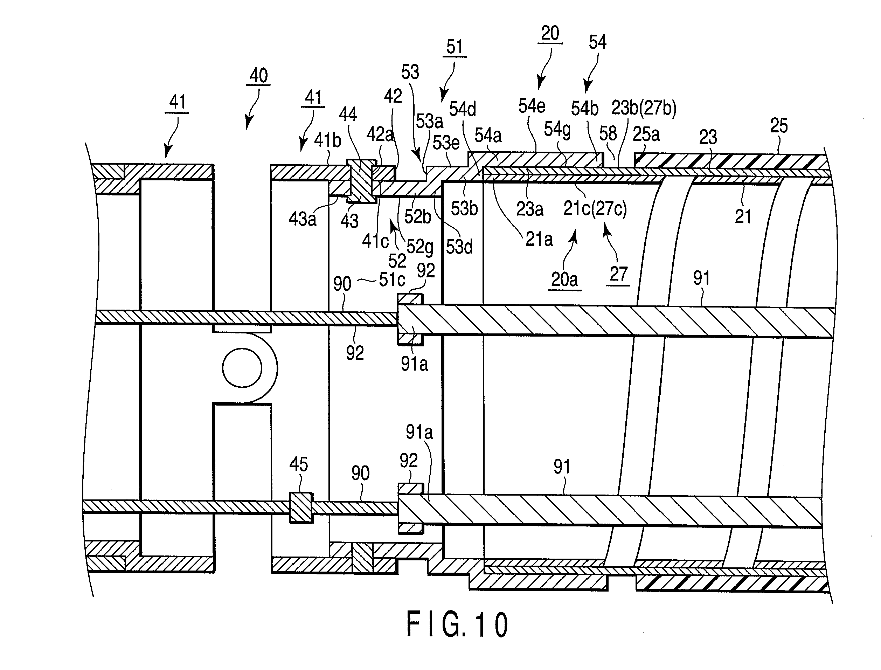

A second embodiment according to the present invention will now be explained with reference to FIG. 10. It should be noted that the same reference numbers as those in the first embodiment denote the same structures as those in the first embodiment, obviating explanation thereof.

Although the distal end 91a is fixed to the inner peripheral surface 27c (inner peripheral surface 21c), i.e., the distal end 20a of the flexible section 20 in the first embodiment, the present invention does not have to be restricted thereto. For example, the distal end 91a may be fixed to the inner peripheral surface 51c of the node ring 51. In more detail, for example, the same excess metal portion 92 as that in the first embodiment is formed on the inner peripheral surface 52g of the distal end portion 52. The distal end 91a is fixed to the inner peripheral surface 52g, i.e., the inner peripheral surface 51c when secured to the excess metal portion 92. In other words, the distal end 91a is fixed to the bending section 40 (node ring 51). It should be noted that the distal end 91a may be fixed by, e.g., soldering or a laser.

It should be noted that the excess metal portion 92 may be formed on the inner peripheral surface 53g of the middle end portion 53 and the distal end 91a may be fixed on the inner peripheral surface 53g. That is, it is good for the distal end 91a to be fixed to the inner peripheral surface 51c of the node ring 51.

As explained above, in this embodiment, the distal end 91a can be fixed to the node ring 51. In this embodiment, a high temperature that is a temperature causing deformation of the envelope 25 can be used when fixing the distal end 91a to the node ring 51 formed of a hard material such as a metal. Therefore, in this embodiment, the distal end 91a can be more firmly fixed. However, this temperature means a temperature at which the node ring 51 is not deformed.

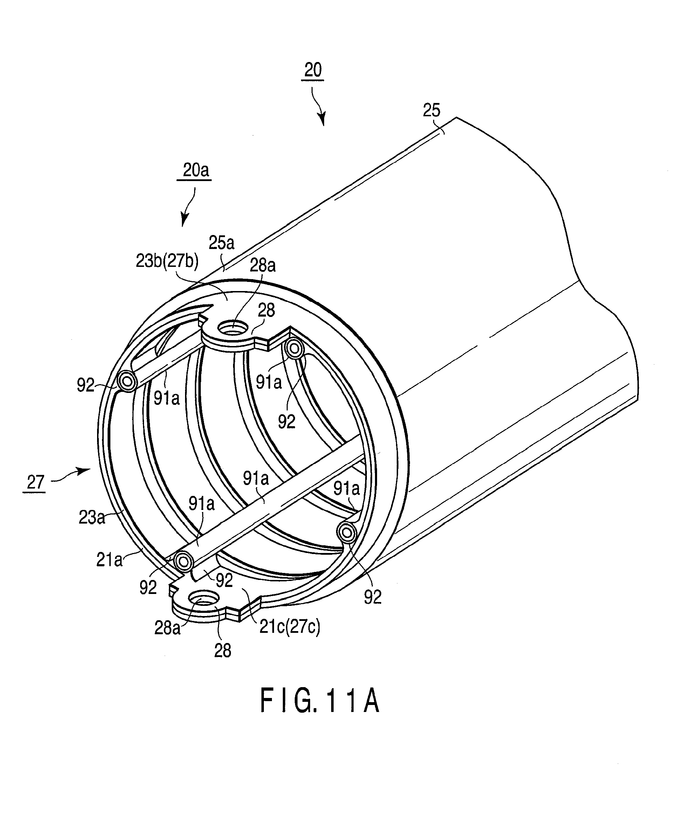

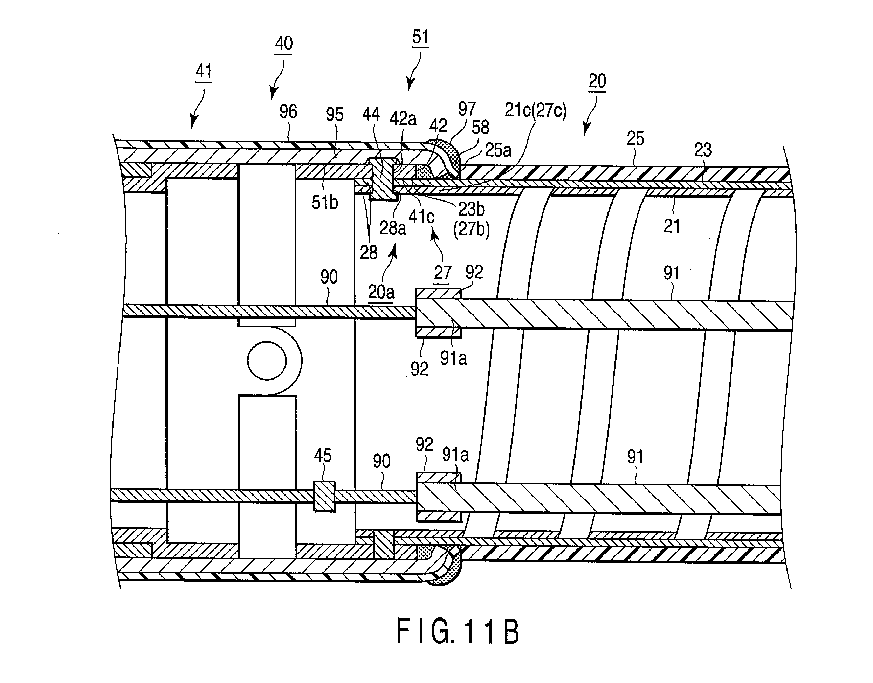

A third embodiment according to the present invention will now be explained with reference to FIGS. 11A and 11B. It should be noted that reference numbers the same as those in the first embodiment denote structures equivalent to those in the first embodiment, obviating explanation thereof.

A flexible section 20 and a bending section 40 in this embodiment are directly coupled to each other to allow them to swivel motion when a flexible tube side hard section 27 is fitted into a node ring 51 and the flexible tube side hard section 27 is coupled to the node ring 51 by spindle sections (e.g., rivets 44 as swiveling members) to allow them to swivel.

As shown in FIG. 11A, in the flexible section 20, a distal end 21a and a distal end 23a are extended toward a distal end 20a side of the flexible section 20 by the desired same length beyond a distal end 25a as in the first embodiment. Therefore, an outer peripheral surface 23b is exposed. Later-explained protruding pieces 42 come into contact with this outer peripheral surface 23b.

Furthermore, as in the first embodiment, the distal end 21a and the distal end 23a are previously secured to be integrated with each other by, e.g., soldering. When the distal end 21a and the distal end 23a are integrated with each other in this manner, the flexible tube side hard section 27 having rigidity is formed.

The flexible tube side hard section 27 (distal end 21a and distal end 23a) in this embodiment is different from the first embodiment. The flexible tube side hard section 27 has two hard protruding pieces 28 formed by partially protruding the flexible tube side hard section 27 (distal end 21a and distal end 23a) toward the front side (node ring 51 side). A hard through-hole 28a is formed in each hard protruding piece 28. The two hard protruding pieces 28 are arranged to be apart from each other in a circumferential direction at substantially 180°. The hard protruding piece 28 is a swiveling coupling portion in the flexible section 20 where the flexible tube side hard section 27 is directly fitted in the node ring 51 and the flexible section 20 is directly coupled to the bending section 40 to allow them to swivel.

Moreover, the node ring 51 according to this embodiment has the same structure as a node ring 41 depicted in FIG. 5. That is, the node ring 51 has two protruding pieces 42 formed by partially protruding the node ring 51 toward the flexible tubes die hard section 27, and a through-hole 42a is formed in each protruding piece 42.

It should be noted that an inner diameter of the node ring 51 is substantially equal to an outer diameter of the flexible tube side hard section 27 (braid 23). Therefore, when the flexible section 20 and the bending section 40 are coupled to each other to allow them to swivel, the flexible tube side hard section 27 is fitted into the node ring 51, and an outer peripheral surface 27b (outer peripheral surface 23b) comes into contact with an inner peripheral surface 41c. The protruding piece 42 is a swiveling coupling portion in the flexible section 40 where the flexible tube side hard section 27 is directly fitted into the node ring 51 and the flexible section 40 is coupled to the flexible section 20 to allow them to swivel. It should be noted that an outer diameter of the node ring 51 is substantially equal to an outer diameter of an envelope 25.

When the flexible section 20 is coupled to the bending section 40 to allow them to swivel, the flexible tube side hard section 27 is fitted into the node ring 51 as shown in FIG. 11B. At this time, the rivets 44 are inserted into the hard through-holes 28a and the through-holes 42a as in the case of coupling the node rings 41 to each other. As a result, the flexible section 20 and the bending section 40 are directly coupled to each other by the rivets 44, allowing them to swivel.

At this time, a surface of each protruding piece 42 comes into contact with the outer peripheral surface 27b (outer peripheral surface 23b) but does not come into contact with the distal end 25a. Therefore, an opening portion 58a is formed between the protruding piece 42 and the distal end 25a as in the first embodiment.

It is preferable for a length of the protruding piece 42 in an insertion direction of an insertion section 10 and a length of the hard protruding piece 28 in the insertion direction of the insertion section 10 to be short. As a result, a length of a non-bent portion can be reduced at a coupling portion for the flexible section 20 and the bending section 40 where they are allowed to swivel.

Coupling the node ring 51 to the flexible section 20 will now be explained.

As in the first embodiment, the flexible tube side hard section 27 is formed at the distal end 20a of the flexible section 20 as shown in FIG. 11. The flexible tube side hard section 27 is directly fitted into the node ring 51 as shown in FIG. 11B. At this time, the outer peripheral surface 27b comes into contact with the inner peripheral surface 41c. In more detail, the hard protruding piece 28 comes into contact with the protruding piece 42. Furthermore, each hard through-hole 28a and each through-hole 42a are arranged on the same straight line in a radial direction.

Moreover, the rivets 44 are inserted into the hard through-holes 28a and the through-holes 42a. As a result, the flexible tube side hard section 27 and the node ring 51 are coupled to each other to allow them to swivel by, e.g., caulking one end of each rivet 44, and they are pivotally supported to allow them to swivel around each rivet 44. In this manner, a spindle section using the rivet 44 as a swiveling spindle is formed between the hard protruding piece 28 and the protruding piece 42.

Additionally, the flexible section 20 and the bending section 40 are directly coupled to each other by the rivets 44 to allow them to swivel. Further, when a distal end 91a is fixed to the flexible tube side hard section 27, the bending section 40 is covered with a braid (mesh tube) 95 as shown in FIG. 11B. This braid 95 covers an outer peripheral surface 51b of the node ring 51. Furthermore, the braid 95 is covered with an envelope 96. Moreover, the braid 95 and the envelope 96 are bonded in the opening portion 58 by, e.g., an adhesive 97 as in the example shown in FIG. 8A.

As a result, in this embodiment, the flexible section 20 and the bending section 40 can be directly coupled to each other by the rivets 44 to allow them to swivel without using a coupling member that couples the flexible section 20 to the bending section 40. That is, in this embodiment, a length of a coupling portion for the flexible section 20 and the bending section 40 in the insertion direction of the insertion section 10 can be reduced, and the coupling portion can swivel.

It should be noted that the hard protruding pieces 28 are provided to the flexible section 20, and the protruding pieces 42 are provided to the node ring 51. The flexible section 20 and the bending section 40 are coupled to each other to allow them to swivel by the hard protruding pieces 28, the protruding pieces 42, and the rivets 44. However, when the flexible section 20 and the bending section 40 can be coupled to each other to allow them to swivel, this embodiment is not restricted to such a conformation.

It is preferable for the protruding piece 42 to be, e.g., arcuate so that the bending section 40 can swivel with respect to the flexible section 20. As a result, in this embodiment, the flexible section 20 and the bending section 40 can be coupled to each other to allow them to swivel even if the protruding pieces 42 are brought into contact with the braid 95.

It should be noted that the protruding piece 42 does not have to be arcuate when the flexible section 20 and the bending section 40 are coupled to each other to allow them to swivel.

Further, the braid 95 and the envelope 96 in this embodiment may be fixed in the opening portion 58 by an adhesive 97 and a thread 98 as in the example shown in FIG. 8B.

It should be noted that the flexible tube side hard section 27 has the flex 21 and the braid 23 in each foregoing embodiment, but the present invention does not have to be restricted thereto.

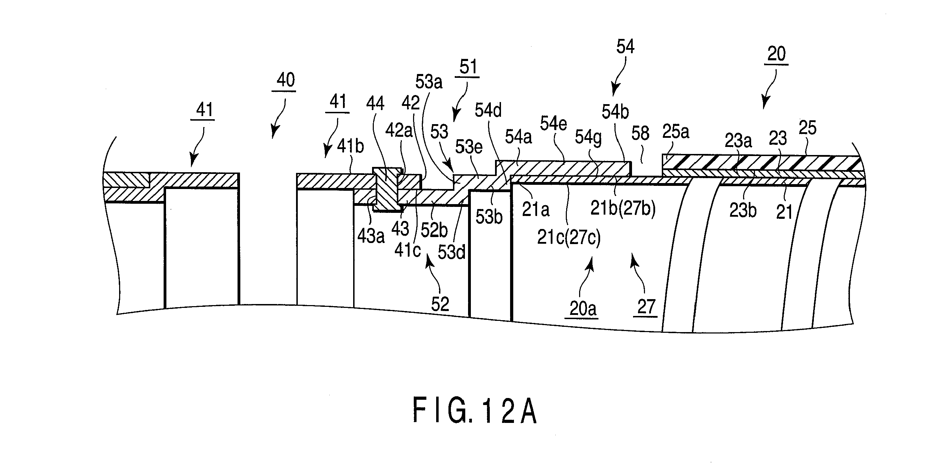

For example, the flexible tube side hard section 27 may be formed of the distal end 21a alone as shown in FIG. 12A. In this case, the distal end 21a is extended to the distal end 20a side of the flexible section 20 beyond the distal end 23a and the distal end 25a. Therefore, the outer peripheral surface 21b and the inner peripheral surface 21c are exposed. In other words, the distal end 23a and the distal end 25a are provided at retired positions as compared with the distal end 21a. It should be noted that the distal end 23a and the distal end 25a are provided at positioned retired from the distal end 21a by the desired same length or different lengths.

Moreover, when the flexible tube side hard section 27 is fitted into the proximal end portion 54, the distal end 21a comes into contact with the step portion 54d, the outer peripheral surface 21b as the outer peripheral surface 27b comes into contact with the inner peripheral surface 54g, and the distal end 21a can be bonded and fixed (coupled) to the proximal end portion 54 as in the above example. As explained above, the flexible tube side hard section 27 may have the flex 21 alone.

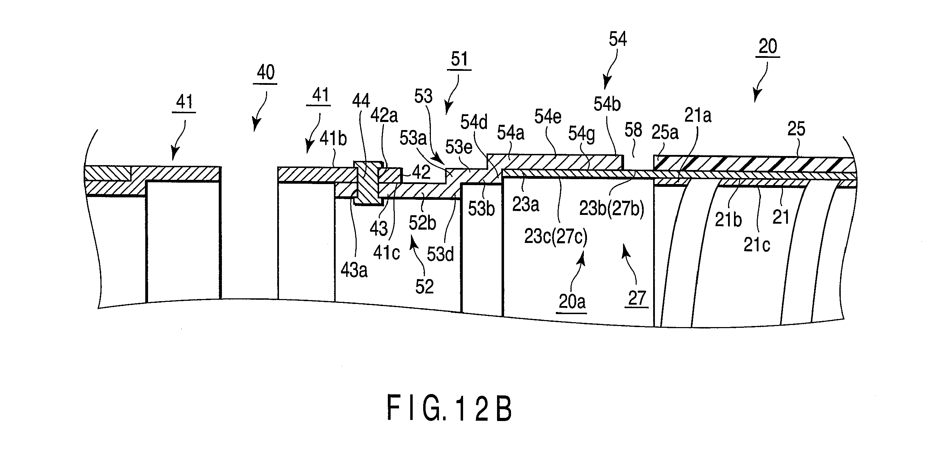

Moreover, for example, the flexible tube side hard section 27 may be formed of the distal end 23a alone as shown in FIG. 12B. In this case, the distal end 23a is extended toward the distal end 20a side of the flexible section 20 beyond the distal end 21a and the distal end 25a. Therefore, the outer peripheral surface 23b and the inner peripheral surface 23c of the distal end 23a are exposed. In other words, the distal end 21a and the distal end 25a are provided at retired positions as compared with the distal end 23a. It should be noted that the distal end 21a and the distal end 25a are provided at positioned retired from the distal end 23a by the desired same length or different lengths.

Further, when the flexible tube side hard section 27 is fitted in the proximal end portion 54, the distal end 23a comes into contact with the step portion 54d, the outer peripheral surface 23b as the outer peripheral surface 27b comes into contact with the inner peripheral surface 54g, and the distal end 23a can be bonded and fixed (coupled) to the proximal end portion 54 as in the above example. As explained above, the flexible tube side hard section 27 may have the braid 23 alone.

It should be noted that a distal end 91a may be fixed to the inner peripheral surface 23c (inner peripheral surface 27c) by an excess metal portion 92 formed on the inner peripheral surface 23c (inner peripheral surface 27c) in this case.

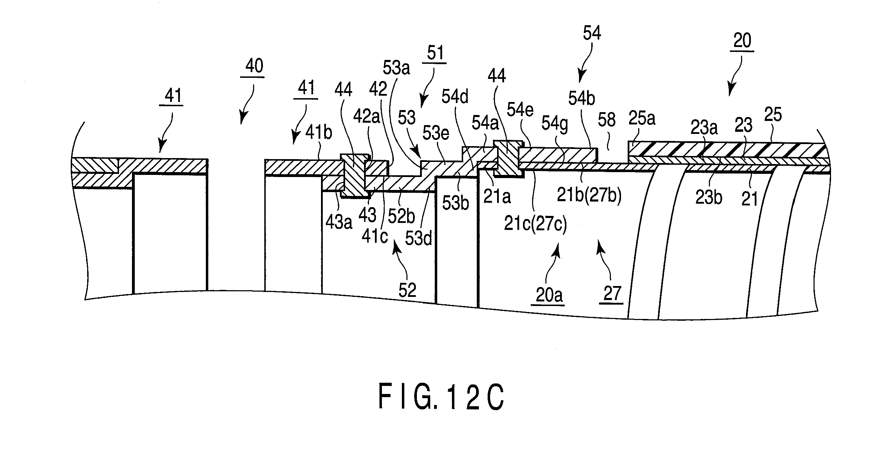

It should be noted that the distal end 21a is bonded and fixed to the proximal end portion 54 in FIG. 12A, but the present invention does not have to be restricted thereto. The distal end 21a a may be coupled to the proximal end portion by a coupling member 46, e.g., a rivet in FIG. 12C.

In the third embodiment according to the present invention, either the hard through-hole 28a or the through-hole 42a may be substituted by a convex portion (not shown) that can be inserted into the other one of the hard through-hole 28a and the through-hole 42a. As a result, in this embodiment, inserting the convex portion into the other hole without using the rivet 44 enables coupling of the flexible section 20 to the bending section 40 to allow them to swivel.

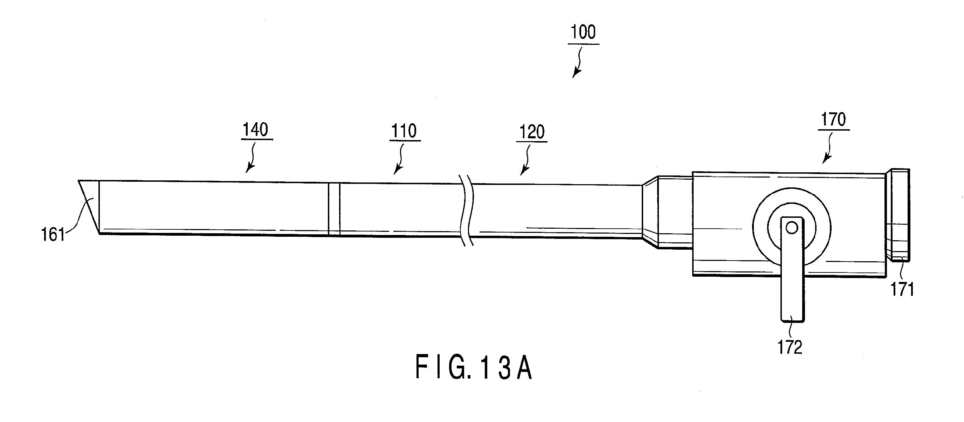

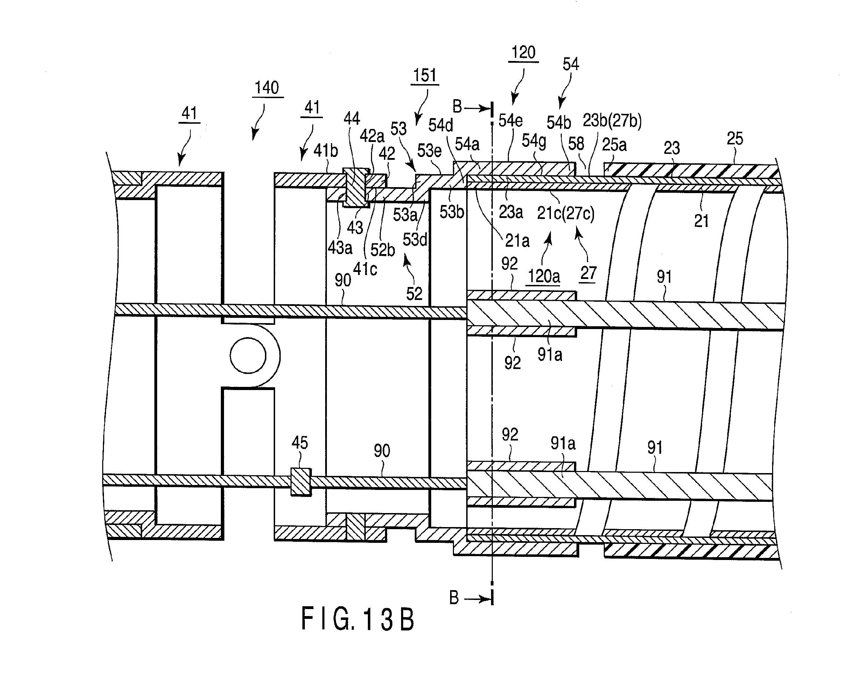

A fourth embodiment according to the present invention will now be explained with reference to FIGS. 13A and 13B. It should be noted that reference numbers to the same as those in the first embodiment denote structures equivalent to those in the first embodiment, obviating explanation thereof.

As shown in FIG. 13A, an endoscope overtube (which will be referred to as an overtube 100 hereinafter) has an elongated insertion section 110 that is inserted into, e.g., a body cavity of a patient and an operating section 170 that is coupled to a proximal end of the insertion section 110 and operates a later-explained bending section 140 of the insertion section 110. An insertion section 10 is inserted into the overtube 100. In more detail, the insertion section 10 is inserted from an insertion opening 171 arranged in the operating section 170 to be protruded from an endoscope outlet 161 in the insertion section 110 through the operating section 170 and the insertion section 110. Each of inner diameters of the insertion opening 171, the operating section 170, a later-explained flexible section 120, a later-explained bending section 140, and the endoscope outlet 161 is larger than an outer diameter of the insertion section 10. The overtube 100 is a guide member that guides the insertion section 10 inserted into the overtube 100 to a desired position in a body cavity.

The insertion section 110 has a the flexible section (corrugated tube section) 120, the bending section 140, and the endoscope outlet 161 in the mentioned order from the operating section 170 side. In more detail, the operating section 170 is coupled to the proximal end of the elongated flexible section 120. A distal end 120a of the flexible section 120 is directly coupled to a proximal end of the bending section 140. In more detail, as shown in FIG. 13B, the flexible section 120 is directly coupled to the bending section 140 with its distal end 120a being fitted in a node ring 151 arranged on the most proximal end side of the bending section 140. A distal end of the bending section 140 is coupled to a proximal end of the endoscope outlet 161.

Since the flexible section 120 has a structure substantially equal to that of the flexible section 20 as shown in FIG. 2, a detailed explanation thereof will be omitted.

Further, since the bending section 140 has a structure substantially equal to that of the bending section 40 as shown in FIG. 2, a detailed explanation thereof will be omitted. It should be noted that a structure of the node ring 151 is substantially equal to that of the node ring 51.

The endoscope outlet 161 is an outlet from which the insertion section 10 inserted into the overtube 100 protrudes. The endoscope outlet 161 is obliquely formed with respect to an axial direction of the overtube 100.

The insertion opening 171 from which the insertion section 10 of the endoscope 1 depicted in FIG. 1 is inserted into the overtube 100 is arranged in the operating section 170. A structure of the operating section 170 other than this opening is substantially equal to that of an operating section 70 of the endoscope 1 depicted in FIG. 1, thereby omitting a detailed explanation thereof. That is, the operating section 170 uses an operation knob 172 to pull operation wires 90 shown in FIG. 13B, thereby bending the bending section 140. As explained above, the operation wires 90 in the operating section 170 (overtube 100) are the same as operation wires 90 in the operating section 70 (endoscope 1).

In this embodiment, since a connection method of the flexible section 120 and the bending section 140 is substantially equal to the connection method of the flexible section 20 and the bending section 40, thus omitting a detailed explanation thereof.

The overtube 100 is inserted into a body cavity in advance. The bending section 140 is bent by an operation of the operation knob 172. In this state, the insertion section 10 is inserted from the insertion opening 171 to be protruded from the endoscope outlet 161 through the operating section 170, the flexible section 120, and the bending section 140.

In this manner, according to this embodiment, the connection method of the flexible section 120 and the bending section 140 is substantially equal to the connection method of the flexible section 20 and the bending section 40. Therefore, this embodiment can obtain the same effects as those in the first to third embodiments in the overtube 100.

Furthermore, this embodiment can improve insertion properties of such an endoscope 1 as shown in FIG. 1 with respect to a body cavity by using such an overtube 100.

Moreover, this embodiment can provide the connection method of the flexible section 120 and the bending section 140 and the production method of the overtube 100 including this connection method.

Additionally, in this embodiment, when inserting the overtube 100 into, e.g., the large intestine, this embodiment enables the coupling portion (node ring 151 and distal end 120a) to smoothly pass through the large intestine or the like, thus reducing patient pain.

Further, this embodiment can provide the overtube 100 at low cost since a coupling member is not used.

Of course, this embodiment can use such structures as depicted in FIGS. 12A, 12B, and 12C.

As explained above, the present invention is not restricted to the foregoing embodiment as it is, and constituent elements can be modified and carried out on an embodying stage without departing from the scope of the invention. Furthermore, appropriately combining a plurality of constituent elements disclosed in the foregoing embodiments enables forming various kinds of inventions.

## Claims

1. An endoscope (1) **characterized by** comprising:
a bending section (40) formed to be bendable by aligning a plurality of substantially cylindrical node rings (41, 51) and coupling the adjoining node rings (41, 51) to each other to allow them to swivel;
a flexible section (20) directly coupled to the bending section (40) by fitting a distal end (20a) thereof into a proximal end side node ring (51) arranged on the most proximal end side of the bending section (40);
an operation wire (90) that is inserted into the flexible section (20) and the bending section (40) and operates the bending section (40); and
a wire guide section (91) that guides the operation wire (90) inserted into the flexible section (20) to the bending section (40) in the flexible section (20).

2. The endoscope (1) according to claim 1, **characterized in that** the flexible section (20) has at the distal end (20a) thereof a flexible tube side hard section (27) that is hard and integrated with the distal end (20a), and the flexible section (20) is directly coupled to the bending section (40) when the flexible tube side hard section (27) is fitted in the proximal end side node ring (51).

3. The endoscope (1) according to claim 2, **characterized in that** an outer diameter of the flexible tube side hard section (27) is substantially equal to an inner diameter of a proximal end portion (54) of the proximal end side node ring (51).

4. The endoscope (1) according to claim 3, **characterized in that** the proximal end side node ring (51) has at the proximal end portion (54) thereof a step portion (54d) with which the flexible tube side hard section (27) comes into contact when the flexible tube side hard section (27) is fitted in the proximal end side node ring (51), and
an outer diameter and an inner diameter of the proximal end portion (54) are larger than an outer diameter of the node ring (41) arranged on a distal end side apart from the proximal end side node ring (51).

5. The endoscope (1) according to claim 4, **characterized in that** the step portion (54d) prevents the flexible tube side hard section (27) from being fitted in to reach the distal end side (52, 53) of the proximal end side node ring (51).

6. The endoscope (1) according to claim 5, **characterized in that**, when the flexible tube side hard section (27) is fitted in the proximal end side node ring (51), the flexible tube side hard section (27) comes into contact with the step portion (54d), an outer peripheral surface (27b) of the flexible tube side hard section (27) comes into contact with an inner peripheral surface (54g) of the proximal end portion (54), and the flexible section (20) is directly coupled to the bending section (40) by coupling the outer peripheral surface (27b) of the flexible tube side hard section (27) to the inner peripheral surface (54g) of the proximal end portion (54).

7. The endoscope (1) according to claim 2, **characterized in that** the flexible section (20) and the bending section (40) are directly coupled to each other to allow them to swivel motion when the flexible tube side hard section (27) is fitted in the proximal end side node ring (51).

8. The endoscope (1) according to claim 7, **characterized in that** the flexible tube side hard section (27) has a pair of hard protruding pieces (28) formed by partially protruding the flexible tube side hard section (27) toward the proximal end side node ring (51), a hard through-hole (28a) being formed in each of the hard protruding pieces (28),
the proximal end side node ring (51) has a pair of protruding pieces (42) formed by partially protruding the proximal end side node ring (51) toward the flexible tube side hard section (27), a through-hole (42a) being formed in each of the protruding pieces (42), and
inserting swiveling members (44) into the through-holes (42a) and the hard through-holes (28a) when the flexible tube side hard section (27) is fitted in the proximal end side node ring (51) enables the flexible section (20) and the bending section (40) to be directly coupled to each other to allow them to swivel.

9. A connection method of a flexible section (20) and a bending section (40) in an endoscope (1) which comprises:
a flexible section (20);
a bending section (40) formed to be bendable by aligning a plurality of substantially cylindrical node rings (41, 51) and coupling the adjoining node rings (41, 51) to each other to allow them to swivel;
an operation wire (90) that is inserted into the flexible section (20) and the bending section (40) and operates the bending section (40); and
a wire guide section (91) that is arranged in the flexible section (20) and guides the operation wire (90) inserted into the flexible section (20) to the bending section (40), the method **characterized by** comprising:
a first step of fitting a distal end (20a, 27) of the flexible section (20) in a proximal end side node ring (51) arranged on the most proximal end side of the bending section (40) to directly couple the bending section (40) and the flexible section (20) to each other; and
a second step of fixing the wire guide section (91) on an inner peripheral surface (27c) of the distal end (27) or an inner peripheral surface (51c) of the proximal end side node ring (51).

10. A production method of an endoscope (1) **characterized by** comprising the connection method of a flexible section (20) and a bending section (40) in an endoscope (1) according to claim 9.

11. An endoscope overtube (100) **characterized by** comprising:
a bending section (140) formed to be bendable by aligning a plurality of substantially cylindrical node rings (41, 151) and coupling the adjoining node rings (41, 151) to each other to allow them to swivel;
a flexible section (120) directly coupled to the bending section (140) by fitting a distal end (120a) thereof into a proximal end side node ring (151) arranged on the most proximal end side of the bending section (140);
an operation wire (90) that is inserted into the flexible section (120) and the bending section (140) and operates the bending section (140); and
a wire guide section (91) that guides the operation wire (90) inserted into the flexible section (120) to the bending section (140) in the flexible section (120).

12. The endoscope overtube (100) according to claim 11, **characterized in that** the flexible section (120) has at the distal end (120a) thereof a flexible tube side hard section (27) that is hard and integrated with the distal end (120a), and the flexible section (120) is directly coupled to the bending section (140) when the flexible tube side hard section (27) is fitted in the proximal end side node ring (151).

13. The endoscope overtube (100) according to claim 12, **characterized in that** an outer diameter of the flexible tube side hard section (27) is substantially equal to an inner diameter of a proximal end portion (54) of the proximal end side node ring (151).

14. The endoscope overtube (100) according to claim 13, **characterized in that** the proximal end side node ring (151) has at the proximal end portion (54) thereof a step portion (54d) with which the flexible tube side hard section (27) comes into contact when the flexible tube side hard section (27) is fitted in the proximal end side node ring (151), and
an outer diameter and an inner diameter of the proximal end portion (54) are larger than an outer diameter of the node ring (41) arranged on a distal end side apart from the proximal end side node ring (151).

15. The endoscope overtube (100) according to claim 14, **characterized in that** the step portion (54d) prevents the flexible tube side hard section (27) from being fitted in to reach the distal end (52, 53) side of the proximal end side node ring (151).

16. The endoscope overtube (100) according to claim 15, **characterized in that**, when the flexible tube side hard section (27) is fitted in the proximal end side node ring (151), the flexible tube side hard section (27) comes into contact with the step portion (54d), an outer peripheral surface (27b) of the flexible tube side hard section (27) comes into contact with an inner peripheral surface (54g) of the proximal end portion (54), and the flexible section (120) is directly coupled to the bending section (140) by coupling the outer peripheral surface (27b) of the flexible tube side hard section (27) to the inner peripheral surface (54g) of the proximal end portion (54).

17. The endoscope overtube (100) according to claim 12, **characterized in that** the flexible section (120) and the bending section (140) are directly coupled to each other to allow them to swivel motion when the flexible tube side hard section (27) is fitted in the proximal end side node ring (151).

18. The endoscope overtube (100) according to claim 17, **characterized in that** the flexible tube side hard section (27) has a pair of hard protruding pieces (28) formed by partially protruding the flexible tube side hard section (27) toward the proximal end side node ring (151), a hard through-hole (28a) being formed in each of the hard protruding pieces (28),
the proximal end side node ring (151) has a pair of protruding pieces (42) formed by partially protruding the proximal end side node ring (151) toward the flexible tube side hard section (27), a through-hole (42a) being formed in each of the protruding pieces (42), and
inserting swiveling members (44) into the through-holes (42a) and the hard through-holes (28a) when the flexible tube side hard section (27) is fitted in the proximal end side node ring (151) enables the flexible section (120) and the bending section (140) to be directly coupled to each other to allow them to swivel.

19. A connection method of a flexible section (120) and a bending section (140) in an endoscope overtube (100) which comprises:
a flexible section (120);
a bending section (140) formed to be bendable by aligning a plurality of substantially cylindrical node rings (41, 151) and coupling the adjoining node rings (41, 151) to each other to allow them to swivel;
an operation wire (90) that is inserted into the flexible section (120) and the bending section (140) and operates the bending section (140); and
a wire guide section (91) that is arranged in the flexible section (120) and guides the operation wire (90) inserted into the flexible section (120) to the bending section (140), the method **characterized by** comprising:
a first step of fitting a distal end (20a, 27) of the flexible section (20) in a proximal end side node ring (151) arranged on the most proximal end side of the bending section (140) to directly couple the bending section (140) and the flexible section (120) to each other; and
a second step of fixing the wire guide section (91) on an inner peripheral surface (27c) of the distal end (27) or an inner peripheral surface (51c) of the proximal end side node ring (151).

20. A production method of an endoscope overtube (100) **characterized by** comprising the connection method of a flexible section (120) and a bending section (140) in an endoscope overtube (100) according to claim 19.
